# EUROPÄISCHE PATENTSCHRIFT

(11) **EP 1 408 948 B1**
(45) Veröffentlichungstag und Bekanntmachung des Hinweises auf die Patenterteilung: **26.09.2007**
(21) Anmeldenummer: 02767050.4
(22) Anmeldetag: 17.07.2002
(51) Int. Cl.: A61K 31/198, A61K 9/20

(54) **SCHILDDRÜSENTHERAPEUTIKA MIT PARTIALGLYCERIDEN UND DEXTRIN**
THYROID THERAPEUTIC AGENT COMPRISING PARTIAL GLYCERIDES AND DEXTRINE
AGENTS THERAPEUTIQUES DE LA GLANDE THYROIDE COMPRENANT DES GLYCERIDES PARTIELS ET UNE DEXTRINE

(30) Priorität: 17.07.2001 DE 10136615
(43) Veröffentlichungstag der Anmeldung: 21.04.2004
(73) Patentinhaber: BERLIN-CHEMIE Aktiengesellschaft, 12489 Berlin (DE)
(72) Erfinder: FLEMMING, Jens, 22041 Hamburg (DE); SCHMITZ, Reinhard, 14055 Berlin (DE); GRÖGER, Karsten, 12587 Berlin (DE); STAUSKE, Karl-Albrecht, 10319 Berlin (DE); ZIMMERMANN, Jens, 12307 Berlin (DE)
(74) Vertreter: Schubert, Klemens
(86) Internationale Anmeldenummer: PCT/DE2002/002673
(87) Internationale Veröffentlichungsnummer: WO 2003/007994

(56) Entgegenhaltungen:
- US-A- 5 562 921
- POUTON C W: "Lipid formulations for oral administration of drugs: non-emulsifying, self-emulsifying and 'self-microemulsifying' drug delivery systems." EUROPEAN JOURNAL OF PHARMACEUTICAL SCIENCES: OFFICIAL JOURNAL OF THE EUROPEAN FEDERATION FOR PHARMACEUTICAL SCIENCES. NETHERLANDS OCT 2000, Bd. 11 Suppl 2, Oktober 2000 (2000-10), Seiten S93-S98, XP002228339 ISSN: 0928-0987
- PATENT ABSTRACTS OF JAPAN vol. 015, no. 017 (C-0796), 14. Januar 1991 (1991-01-14) & JP 02 264721 A (TAMA SEIKAGAKU KK), 29. Oktober 1990 (1990-10-29)

## Beschreibung

Die Erfindung betrifft orale Schilddrüsentherapeutika, enthaltend mindestens einen Wirkstoff und als Hilfsstoffe Dextrine und höherkettige Partialglyceride, wobei einer der Wirkstoffe Levothyroxin oder dessen pharmazeutisch verträgliche Salze oder Liothyronin oder dessen pharmazeutisch verträgliche Salze oder Mischungen aus der genannten Wirkstoffen sind.

Die zeitabhängige Verteilung von Arzneistoffen und deren Metabolite im (menschlichen) Organismus ist Untersuchungsgegenstand der Pharmakokinetik.

Zur (quantitativen) Beschreibung des zeitlichen Verlaufs der Arzneistoff- bzw. Metabolitenkonzentration im Organismus werden pharmakokinetische Modelle herangezogen bzw. entwickelt, die es gestatten sollen, die Arzneistoffkonzentration zu jedem beliebigen Zeitpunkt nach der Applikation zu berechnen bzw. vorherzusagen, um so auch zu optimalen Dosierungs- bzw. Anwendungsrichtlinien für Arzt und Patient zu gelangen. Wichtige Parameter zur Beschreibung von (Blutspiegel-) Konzentrations-Zeit-Kurven sind die Fläche unter der Kurve, AUC, das Konzentrationsmaximum, Cₘₐₓ, und der Zeitpunkt der maximalen Konzentration, tₘₐₓ, des Arzneistoffes im Blut (Derendorf, H., Garrett, E. R., Pharmakokinetik, Wiss. Verlagsgesell. Stuttgart, 1987).

Zur Prüfung der (therapeutischen) Gleichwertigkeit von den gleichen Arzneistoff enthaltenden (und auch galenisch vergleichbaren) Darreichungsformen werden Bioverfügbarkeits- bzw. Bioäqivalenzuntersuchungen durchgeführt.

Gemäß den geltenden Richtlinien kann unter bestimmten Umständen auf kostenintensive Bioverfügbarkeits- bzw. Bioäquivalenzuntersuchungen bei mit unterschiedlicher Dosierungsstärken des Arzneistoffes vorliegenden (gleichen) Darreichungsformen verzichtet werden. Dies insbesondere dann, wenn die Pharmakokinetik des Arzneistoffes linear ist (EU-Guideline 3CC15a: Investigation of Bioavailability and Bioequivalance, Dezember 1991, CPMP/EWP/QWP/1401/98 - Draft: Note for Guidance on the Investigation of Bioavailability and Bioequivalence).

In der Literatur werden für verschiedene Arzneistoffe eine zur Dosis lineare Pharmakokinetik (Dosislinearität) beschrieben.

Unter dem Begriff "Dosislinearität" wird dabei im allgemeinen verstanden, dass sich der Anstieg der (Blutspiegel-) Konzentration eines Arzneistoffes direkt proportional zur verabreichten Dosis verhält. Aus pharmakokinetischer Sicht ist bei einer linearen Kinetik der nach Verabreichung verschiedener Dosierungen eines Arzneistoffes aus den (Blutspiegel-) Konzentrations-Zeit-Kurven ermittelte Parameter AUC beispielsweise nach (linearer) Normierung zwischen den Dosen nicht mehr (signifikant) unterscheidbar.

Mason, W. D., Winer, N. und Kochak, G. et al. (Clin. Pharmacol. Ther. 25, 806, 1979) fanden für den Betarezeptorenblocker Atenolol eine lineare Pharmakokinetik.
Für Chinin wurde eine Dosislinearität von Babalola, C. P., Bolaji, O. O. und Ogunbona, F. A. et al. (Eur. J. Pharm. Biopharm. 44, 143, 1997) beobachtet.
Eine lineare Pharmakokinetik für den Arzneistoff Brodimoprin wiesen Acerbi, D., Ventura, P. und Deroubaix, X. et al. (Eur. J. Drug Metab. Pharmacokinet. 1993, Spec. Issue, Proceedings of the 5th European Congress of Biopharmaceutics and Pharmacokinetics) nach.

Für Chlortalidon konnte ebenfalls eine Linearität im Dosierungsbereich von ≤ 100 mg durch Riess, W., Dubach, U. C. und Burckhardt, D. et al. (Eur. J. Clin. Pharmacol. 12, 375, 1977) sowie Fleuren, H. L. J., Wissen, C. V. und van Rossum, J. M. et al. (Clin. Pharmacol. Ther. 25, 806, 1979) beobachtet werden.

Für das Antidiabetikum Glimepirid bestätigten Malerczyk, V., Badian, M., Korn A. et al. (Drug Metab. Drug Interact. 11, 341, 1994) im Dosierungsbereich zwischen 1 und 8 mg eine Dosislinearität.

Für das in der Therapie von Schilddrüsenerkrankungen eingesetzte Levothyroxin bzw. sein Salz Levothyroxin-Natrium ist in der Literatur derzeit keine lineare Pharmakokinetik beschrieben bzw. nachgewiesen.

Im Gegenteil, Biersack, H.-J., Breul, H. P., Rupp, K. P. und Niemeyer, M. (Nuklearmedizin 2, A52, 2000) stellten in ihrer Veröffentlichung über "Untersuchungen zur pharmakokinetischen Linearität von Levo-Thyroxin nach oraler Verabfolgung bei Patienten unter Langzeit-Medikation bei L-Thyroxin" fest, " dass sowohl die L-Thyroxin-Serumkonzentration als auch die aus ihnen berechneten pharmakokinetischen Parameter (area under curve (AUC) 0 x 24, Cₘₐₓ, C_{trough} (jeweils im Steady State nach 6 Wochen Medikation) einer nicht-linearen Kinetik unterliegen".

Aus der WO 98/18610 ist die Verwendung von Dextrinen und höherkettigen Partialglyceriden zur Herstellung von eingebetteten und verkapselten Teilchen mit kontrollierter in-vitro Freisetzung bekannt. Dabei wird die Matrix durch Wärme geschmolzen und plastifiziert, abgekühlt und mit den Wirkstoffen vermischt und extrudiert. In seltenen Fällen werden dabei Cyclodextrine und Dextrine verwendet. Hydrophobe Stoffe, u. a. auch Partialglyceride, können zur Kontrolle der Freisetzung verwendet werden. Es entsteht eine pastöse Masse, die in diskrete Partikel, Pellets oder Tabletten geformt wird. Die Verwendung von Tablettierhilfsstoffen wie Calciumphosphat und Cellulose werden nicht beschrieben.

Pouton, C. W. (Eur. J. Pharm. Sci., 11 Suppl. 2, S93-S98) beschreibt Lipidformulierungen, welche neben Triglyceriden oder gemischten Glyceriden Tenside enthalten und Lipidfreisetzungssysteme darstellen. Je nach Tensid oder bei Anwesenheit hydrophiler Co-Lösemittel werden Systeme gebildet, welche selbst emulgierend sind. Die gleichzeitige Verwendung von Dextrinen und höherkettigen Partialglyceriden wird nicht offenbart.

In der US-A 5,562,921 werden stabile feste pharmazeutische Zusammensetzungen beschrieben, welche Enalaprilmaleat als Wirkstoff enthalten. Hierin wird die Verwendung von Dextrin als wasserlöslichem Bestandteil der Formulierung und die Verwendung von Glycerinmonostearat als Gleitmittel offenbart.

In der JP-A 02 264721 wird eine Vitamin E-Zubereitung offenbart, welche einen Emulgator wie Glycerinfettsäureester und Dextrin enthält. Weiterhin enthält die Zubereitung noch Casein und Gummi arabicum, um eine leichte Dispersibilität des Vitamin E in Wasser zu bewirken.

Aufgabe der vorliegenden Erfindung ist es, Schilddrüsentherapeutika zur Verfügung zu stellen, welche die Nachteile des Standes der Technik überwinden.

Die Aufgabe wird dadurch gelöst, dass ein orales Schilddrüsentherapeutikum, enthaltend mindestens einen Wirkstoff und als Hilfsstoffe Dextrine und höherkettige Partialglyceride sowie weitere pharmazeutisch annehmbare Hilfs- und Trägerstoffe zur Verfügung gestellt wird, wobei einer der Wirkstoffe Levothyroxin oder dessen pharmazeutisch verträgliche Salze oder Liothyroxin oder dessen pharmazeutisch verträgliche Salze oder Mischungen aus der genannten Wirkstoffen sind.

Die Aufgabe wird dadurch gelöst, dass orale Schilddrüsentherapeutikua durch einfaches Mischen eines oder mehrerer Wirkstoffe (z.B. Levothyroxin-Natrium etc.) mit den Hilfsstoffen Dextrin und höherkettigen Partialglyceriden sowie mit weiteren pharmazeutisch annehmbaren Hilfs- und Trägerstoffen, vorzugsweise Calciumphosphat und Cellulose, und anschließendes Pressen zu Tabletten hergestellt werden. Denkbar ist auch das direkte Befüllen von Kapseln mit der Basisrezeptur oder aus der Basisrezeptur hergestellten (Mikro-) Tabletten.

überraschenderweise wurde gefunden, dass erfindungsgemäße Schilddrüsentherapeutika, welche neben den Wirkstoffen Dextrine und höherkettige Partialglyceride enthalten, eine Freisetzungscharakteristik besitzen, welche unabhängig von der jeweiligen Dosierungsstärke des Wirkstoffes ist. Außerdem ist die in-vivo Verfügbarkeit proportional zur verabreichten Dosis, d.h. eine Dosislinearität wird erreicht. Die Dosierungsstärke wird erfindungsgemäß nur durch eine Variation der Tablettenmasse bewirkt, wobei das zur Einzeldosierung verarbeitete Basisgranulat für alle Dosisstärken identisch ist.

Erfindungsgemäß bevorzugt ist es, dass der Gehalt an Dextrinen 5 bis 60 Gew.-% beträgt. Erfindungsgemäß besonders bevorzugt ist es dabei, dass der Gehalt an Dextrinen 15 bis 50 Gew.-% beträgt.

Erfindungsgemäß bevorzugt ist es auch, dass der Gehalt an höherkettigen Partialglyceriden 0,1 bis 10 Gew.-% beträgt. Besonders bevorzugt ist es dabei, dass der Gehalt an höherkettigen Partialglyceriden 0,5 bis 4 Gew.-% beträgt.

Die erfindungsgemäß verwendeten Dextrine und höherkettigen Partialglyceride sind dem Fachmann bekannt. Dextrin ist ein Gemisch von Polysacchariden, das durch Teilhydrolyse von Stärke gewonnen wird. Höherkettige Partialglyceride bestehen aus einem Gemisch aus Mono-, Di-und Triglyceriden gesättigter Fettsäuren natürlichen Ursprungs, hauptsächlich der Hexadecansäure und der Octadecansäure. Beide Stoffe sind in den Arzneibüchern beschrieben (DAB 1999, NF 19).

Ganz besonders ist es erfindungsgemäß bevorzugt, dass weiterhin als Hilfsstoff Calciumphosphat enthalten ist. Calciumphosphate und insbesondere bevorzugt Calciumhydrogenphosphat bewirken, dass die Mischung sich gut zu Tabletten verpressen lässt. Es entstehen ausreichend harte Formlinge. Im Gegensatz zu Extrusionsverfahren tritt bei der Verwendung keine Krümelbildung auf. Besonderes bevorzugt sind hierbei Anteile an diesem Hilfsstoff von 10 bis 45 Gew.-%.

Weiterhin ist ganz besonders bevorzugt, dass weiterhin als Hilfsstoff Cellulose enthalten ist. Geeignete Cellulose ist insbesondere Mikrokristalline Cellulose. Besonders bevorzugt sind dabei Anteile von 10 bis 65 Gew.-% an diesem Hilfsstoff.

Bevorzugte erfindungsgemäße orale Schilddrüsentherapeutika sind dadurch gekennzeichnet, dass der Wirkstoffgehalt 0,01 bis 5 Gew. -% beträgt. Dabei ist es bevorzugt, dass der Wirkstoffgehalt 0,05 bis 2,5 Gew.-% beträgt. Besonders bevorzugt ist es, dass der Wirkstoffgehalt 0,1 bis 1 Gew.-% beträgt.

Ein weiterer Gegenstand der vorliegenden Erfindung sind auch orale Schilddrüsentherapeutika, welche als weiteren Wirkstoff Iodsalze enthalten. Erfindungsgemäß besonders bevorzugt ist dabei, dass die Iodsalze Iodide oder Iodate der Alkalimetalle sowie deren Mischungen, insbesondere Kaliumiodid oder Kaliumiodat sind.

Ganz besonders bevorzugt ist ein erfindungsgemäßes orales Schilddrüsentherapeutikum, enthaltend neben weiteren pharmazeutisch annehmbaren Hilfs- und Trägerstoffen, 5 bis 60 Gew.-% Dextrine, 0,1 bis 10 Gew.-% höherkettige Partialglyceride, 0,01 bis 5 Gew.-% Levothyroxin und/oder Liothyronin oder deren pharmazeutisch verträgliche Salze, 10 bis 45 Gew.-% Calciumhydrogenphosphat und 10 bis 65 Gew.-% Mikrokristalline Cellulose.

Erfindungsgemäß ist es auch, dass das orale Schilddrüsentherapeutikum in Form einer mit Basisrezeptur oder mit (Mikro-) Tabletten gefüllten Kapsel, einer einfach oder mehrfach teilbaren Tablette vorliegt. Besonders bevorzugt sind dabei teilbare Tabletten, die eine Bruchrille, mehrere parallel zueinander angeordnete Bruchrillen oder eine Kreuzbruchrille aufweisen.

Die vorliegende Erfindung betrifft somit ein Konzept und Verfahren zur Herstellung eines Schilddrüsentherapeutikum in verschiedenen Dosierungsstärken aus einem gemeinsamen Grund- bzw. Basisgranulat, um eine in Abhängigkeit von der gegebenen Dosis lineare Pharmakokinetik für das enthaltene Schilddrüsenhormon nach dessen oraler Verabreichung zu erhalten.

Überraschenderweise wurde nun für Levothyroxin-Natrium eine Dosislinearität nach oraler Verabreichung von Tabletten unterschiedlicher Dosierungen gefunden, wobei die unterschiedlichen Dosierungen des Arzneistoffes Levothyroxin-Natrium allein durch Variation der Masse der Tabletten aus einer einheitlichen Grundrezeptur realisiert wurden.

Alle unterschiedlich dosierten Tabletten wurden aus einem Levothyroxin-Natrium haltigen Grund- bzw. Basisgranulat durch Variation der Tablettenmasse hergestellt, wodurch deren prozentuale Zusammensetzung unverändert blieb. Die Zusammensetzung des verwendeten Basisgranulates ist in Beispiel 1 angegeben.

Gegenstand der vorliegenden Erfindung sind Arzneimittel zur oralen Applikation, die neben üblichen Träger- und Verdünnungsmitteln einen oder mehrere Wirkstoffe enthalten.

Die Herstellung der erfindungsgemäßen oralen pharmazeutischen Zubereitungen ist an sich bekannt und in den dem Fachmann bekannten Handbüchern beschrieben, beispielsweise Hager's Handbuch (5.) 2, 622-1045; List et al., Arzneiformenlehre, Stuttgart: Wiss. Verlagsges. 1985; Sucker et al., Pharmazeutische Technologie, Stuttgart: Thieme 1991; Ullmann's Enzyklopädie (5.) A 19, 241-271; Voigt, Pharmazeutische Technologie, Berlin: Ullstein Mosby 1995.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können auch Mikrotabletten hergestellt werden, welche dann in Kapseln eingefüllt werden können. Aber auch das Pulver/Granulat der Basisrezeptur kann direkt in Kapseln abgefüllt werden. Mit Hilfe üblicher Kapselfüllmaschinen ist es daher möglich, verschieden dosierte Präparate ohne große Umrüstzeiten herzustellen, da stets die identische Basisrezeptur verwendet wird. Es ist lediglich eine Einstellung der Füllmenge erforderlich, um anders dosieret Formen herzustellen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Auch die Herstellung von Tabletten mit Bruchrillen ist dem Fachmann an sich bekannt. Hierbei werden entsprechend ausgestaltete Presswerkzeuge verwendet, und die Tabletten mit den erforderlichen Bruchrillen zu versehen. Dabei können Kreuzbruchrillen ebenso angebracht werden, wie Bruchrillen und mehrfache Bruchrillen, wobei dann gegebenenfalls von der runden Tabellenform zu einer länglichen Form übergegangen werden kann.

Die folgenden Beispiele erläutern die Erfindung:

Die erfindungsgemäßen Basisrezepturen weisen typischerweise die folgenden Zusammensetzungen auf:

### Beispiel 1A

### Zusammensetzung der Basisrezeptur (Levothyroxin-Natrium)

| | |
|---|---|
| Levothyroxin-Natrium | 0,01 - 1 % |
| Dextrin | 0,1 - 99 % |
| Partialglyceride | 0,1 - 10 % |
| Tablettierhilfsstoffe | ad 100 % |

### Beispiel 1B

### Zusammensetzung der Basisrezeptur (Liothyronin-Natrium)

| | |
|---|---|
| Liothyronin-Natrium | 0,01 - 1 % |
| Dextrin | 0,1 - 99 % |
| Partialglyceride | 0,1 - 10 % |
| Tablettierhilfsstoffe | ad 100 % |

### Beispiel 1C

### Zusammensetzung der Basisrezeptur (Levothyroxin-Natrium und weiterer Wirkstoff)

| | |
|---|---|
| Levothyroxin-Natrium | 0,01 - 1 % |
| weiterer Wirkstoff | 0,01 - 10 % |
| Dextrin. | 0,1 - 99 % |
| Partialglyceride | 0,1 - 10 % |
| Tablettierhilfsstoffe | ad 100 % |

### Beispiel 1D

### Zusammensetzung de Basisrezeptur (Liothyronin-Natrium und weiterer Wirkstoff)

| | |
|---|---|
| Liothyronin-Natrium | 0,01 - 1 % |
| weiterer Wirkstoff | 0,01 - 10 % |
| Dextrin | 0,1 - 99 % |
| Partialglyceride | 0,1 - 10 % |
| Tablettierhilfsstoffe | ad 100 % |

### Beispiel 2:

### Zusammensetzung der Basisrezeptur in [%] für die Herstellung verschieden dosierter Tabletten :

| | |
|---|---|
| Levothyroxin-Natrium | 0,09 % |
| Calciumhydrogenphosphat-Dihydrat | 28,32 % |
| Mikrokristalline Cellulose | 31,59 % |
| Carboxymethylstärke-Natrium, Typ A | 24,87 % |
| Dextrin | 12,68 % |
| höherkettige Partialglyceride | 2,45 % |

Unter Verwendung dieser Basisrezeptur ist es möglich, allein durch Variation der Tablettenmasse unterschiedlich dosierte Tabletten herzustellen, wobei deren prozentuale Zusammensetzung und somit das Verhältnis von Levothyroxin-Natrium und Hilfsstoffen unverändert bleibt. Eine mögliche Auswahl der mit Hilfe dieses Granulates herstellbaren Dosierungen ist in Beispiel 3 aufgeführt, wobei weitere Dosierungen denkbar sind.

### Beispiel 3:

Herstellung von unterschiedlich dosierten Levothyroxin-Natrium haltigen Tabletten unter Nutzung einer Basisrezeptur allein durch Variation der Tablettenmasse (Auswahl). Hierfür wurde das Granulat gemäß Beispiel 2 verwendet.

| | |
|---|---|
| Dosierung 50 µg* | Tablettenmasse 52 mg |
| Dosierung 75 µg* | Tablettenmasse 78 mg |
| Dosierung 100 µg* | Tablettenmasse 104 mg |
| Dosierung 125 µg* | Tablettenmasse 130 mg |
| Dosierung 150 µg* | Tablettenmasse 156 mg |
| Dosierung 175 µg* | Tablettenmasse 182 mg |
| Dosierung 200 µg* | Tablettenmasse 208 mg |
| Dosierung 300 µg* | Tablettenmasse 312 mg |

| | |
|---|---|
| * Sollgehalt Levothyroxin-Natrium | |

Für die unter Nutzung der beschriebenen Basisrezeptur hergestellten Tabletten wurde in Auswertung der Bioverfügbarkeitsuntersuchungen überraschenderweise im Gegensatz zu den bisherigen Angaben in der Literatur eine klare Dosislinearität für Levothyroxin-Natrium erhalten. Die ermittelten pharmakokinetischen Parameter der in vivo geprüften 50, 100, 150 und 200 µg Levothyroxin-Natrium enthaltenden Tabletten sind in Tabelle 1 aufgeführt.

**Tabelle 1:**

| Aus Bioverfügbarkeitsuntersuchungen ermittelte pharmakokinetische Parameter der geprüften Levothyroxin-Natrium Tabletten | | |
|---|---|---|
| Dosierungsform | AUC₀₋₂₄^{*,**} | C_{max, 0-24}^{**} |
| 50 µg | 3381 | 147,5 |
| 100 µg | 4370 | 204,3 |
| 150 µg | 5268 | 252,8 |
| 200 µg | 6239 | 309,9 |

| | | |
|---|---|---|
| * Angabe für T4, repeated dosing ** Angabe in [nmol*h/l] | | |

### Beispiel 4:

Nachfolgend werden weitere erfindungsgemäße Zusammensetzungen für Basisrezepturen angegeben:

### Beispiel 4A:

| | |
|---|---|
| Levothyroxin-Natrium | 0,08% |
| Dextrin | 20 % |
| Partialglyceride | 1,5 % |
| Mikrokristalline Cellulose | 40 % |
| Calciumhydrogenphosphat | 23,42% |
| Maisstärke | 15 % |

### Beispiel 4B:

| | |
|---|---|
| Levothyroxin-Natrium | 0,1 % |
| Dextrin | 15 % |
| Partialglyceride | 2,5 % |
| Mikrokristalline Cellulose | 25 % |
| Calciumhydrogenphosphat | 40 % |
| Carboxymethylstärke-Natrium, Typ | A 17,4 % |

### Beispiel 4C:

| | |
|---|---|
| Levothyroxin-Natrium | 0,1 % |
| Kaliumiodid | 0,1 % |
| Dextrin | 25 % |
| Partialglyceride | 0,5 % |
| Mannitol | 30 % |
| Calciumhydrogenphosphat | 30 % |
| Gelatine | 2,5 % |
| Carboxymethylstärke-Natrium, Typ A Magnesiumstearat | 10,8 % 1 % |

### Beispiel 4D:

| | |
|---|---|
| Levothyroxin-Natrium | 0,05% |
| Kaliumiodid | 0,2 % |
| Dextrin | 50 % |
| Partialglyceride | 4 % |
| Mikrokristalline Celulose | 36 % |
| Polyvinylpyrolidon | 5 % |
| Ethylcellulose | 2 % |
| Talk | 2,5 % |
| Silikonemulsion | 0,25% |

### Beispiel 4E:

| | |
|---|---|
| Liothyronin-Natrium | 0,25% |
| Dextrin | 30 % |
| Partialglyceride | 1,5 % |
| Mikrokristalline Cellulose | 55 % |
| Carboxymethylstärke-Natrium, Typ A | 12 % |
| Stearinsäure | 1,25% |

### Beispiel 5:

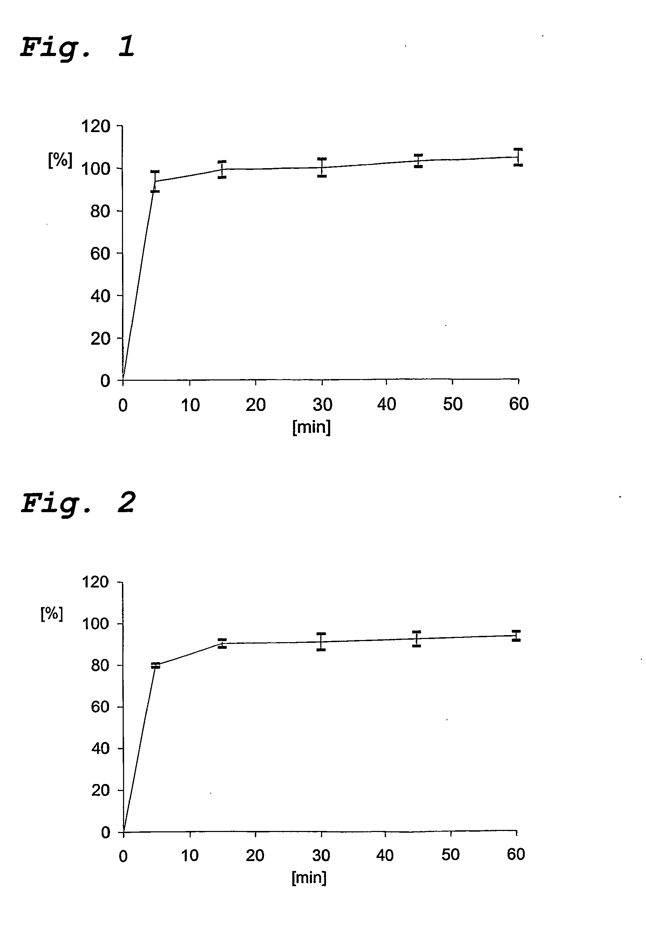

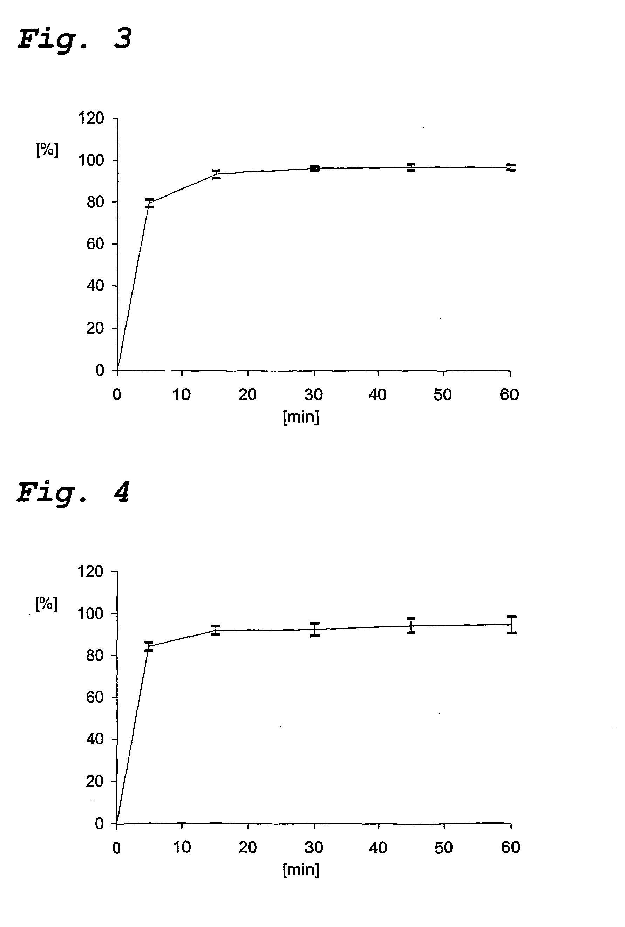

Die folgenden in vitro Untersuchungen der erfindungsgemäß hergestellten Tabletten belegen, dass der Wirkstoff Levothyroxin-Natrium aus den Tabletten der unterschiedlichen Dosierungen in gleicher Geschwindigkeit und gleichem Ausmaß freigesetzt und somit dem Körper zur Verfügung gestellt werden kann. Die Untersuchungen zur Freisetzung wurden mit den Tabletten gemäß den Beispielen 2 und 3 durchgeführt. In den Figuren 1 bis 4 ist die jeweilige Freisetzung dargestellt. An der y-Achse ist die freigesetzte Menge in % angegeben, an der x-Achse ist die Zeit aufgetragen. Die Balken geben den Vertrauensbereich des Mittelwerts der Messungen an.

Es zeigen:
Fig. 1: Freisetzung von Levothyroxin-Natrium aus Tabletten (50 µg), Tablettenmasse 52 mg, Paddle-Apparatur, 100 rpm, Freisetzungsmedium: 500 ml Phosphatpuffer pH 7,4;
Fig. 2: Freisetzung von Levothyroxin-Natrium aus Tabletten (100 µg), Tablettenmasse 104 mg, Paddle-Apparatur, 100 rpm, Freisetzungsmedium: 500 ml Phosphatpuffer pH 7, 4;
Fig. 3: Freisetzung von Levothyroxin-Natrium aus Tabletten (150 µg), Tablettenmasse 156 mg, Paddle-Apparatur, 100 rpm, Freisetzungsmedium: 500 ml Phosphatpuffer pH 7,4 und
Fig. 4: Freisetzung von Levothyroxin-Natrium aus Tabletten (200 µg), Tablettenmasse 208 mg, Paddle-Apparatur, 100 rpm, Freisetzungsmedium: 500 ml Phosphatpuffer pH 7,4.

Diese Untersuchungen zeigen ferner, dass die Tabletten problemlos geteilt werden können, da gleichzeitig beispielsweise mit einer Halbierung oder Viertelung der Tabletten die Dosis des oder der Wirkstoffe entsprechend halbiert bzw. geviertelt wird. Dabei wird kein Einfluss auf die Dosierung und die Bioverfügbarkeit beobachtet, da sich die Freisetzung nicht ändert.

Durch die vorliegende Erfindung wird es möglich, für alle innerhalb des nachgewiesenen Linearitätsbereichs liegenden (denkbaren) Dosierungsstärken auf kostenintensive Bioverfügbarkeits- und Bioäquivalenzuntersuchungen zu verzichten, insbesondere ein individuell auf den Patienten ausgerichtetes Dosierungsschema zu realisieren sowie die bisher üblichen, regelmäßigen Kontrolluntersuchungen von Patienten mit einer L-Thyroxin-Medikation einzuschränken.

## Patentansprüche

1. Orales Schilddrüsentherapeutikum, enthaltend mindestens einen Wirkstoff und als Hilfsstoffe Dextrine und höherkettige Partialglyceride, gemäß den Arzneibüchern DAB 1999 oder NF 19, sowie weitere pharmazeutisch annehmbare Hilfs- und Trägerstoffe, wobei einer der Wirkstoffe Levothyroxin oder dessen pharmazeutisch verträgliche Salze oder Liothyronin oder dessen pharmazeutisch verträgliche Salze oder Mischungen aus den genannten Wirkstoffen sind.

2. Orales Schilddrüsentherapeutikum gemäß Anspruch 1, **dadurch gekennzeichnet, dass** der Gehalt an Dextrinen 5 bis 60 Gew.-% beträgt.

3. Orales Schilddrüsentherapeutikum gemäß einem der voranstehenden Ansprüche, **dadurch gekennzeichnet, dass** der Gehalt an Dextrinen 15 bis 50 Gew.-% beträgt.

4. Orales Schilddrüsentherapeutikum gemäß einem der voranstehenden Ansprüche, **dadurch gekennzeichnet, dass** der Gehalt an höherkettigen Partialglyceriden 0,1 bis 10 Gew.-% beträgt.

5. Orales Schilddrüsentherapeutikum gemäß einem der voranstehenden Ansprüche, **dadurch gekennzeichnet, dass** der Gehalt an höherkettigen Partialglyceriden 0,5 bis 4 Gew.-% beträgt.

6. Orales Schilddrüsentherapeutikum gemäß einem der voranstehenden Ansprüche, **dadurch gekennzeichnet, dass** weiterhin als Hilfsstoff Calciumphosphat enthalten ist.

7. Orales Schilddrüsentherapeutikum gemäß einem der voranstehenden Ansprüche, **dadurch gekennzeichnet, dass** weiterhin als Hilfsstoff Cellulose enthalten ist.

8. Orales Schilddrüsentherapeutikum gemäß einem der voranstehenden Ansprüche, **dadurch gekennzeichnet, dass** der Wirkstoffgehalt 0,01 bis 5 Gew.-% beträgt.

9. Orales Schilddrüsentherapeutikum gemäß Anspruch 1, **dadurch gekennzeichnet, dass** der Wirkstoffgehalt 0,05 bis 2,5 Gew.-% beträgt.

10. Orales Schilddrüsentherapeutikum gemäß Anspruch 1, **dadurch gekennzeichnet, dass** der Wirkstoffgehalt 0,1 bis 1 Gew.-% beträgt.

11. Orales Schilddrüsentherapeutikum gemäß einem der voranstehenden Ansprüche, **dadurch gekennzeichnet, dass** einer der Wirkstoffe ein Schilddrüsenhormon ist.

12. Orales Schilddrüsentherapeutikum gemäß einem der voranstehenden Ansprüche, **dadurch gekennzeichnet, dass** ein weiterer Wirkstoff Iodsalze sind.

13. Orales Schilddrüsentherapeutikum gemäß Anspruch 12, **dadurch gekennzeichnet, dass** die Iodsalze Iodide oder Iodate der Alkalimetalle sowie deren Mischungen, insbesondere Kaliumiodid oder Kaliumiodat sind.

14. Orales Schilddrüsentherapeutikum gemäß einem der voranstehenden Ansprüche, in Form einer Kapsel, nicht-, einfach oder mehrfach teilbaren Tablette.

15. Orales Schilddrüsentherapeutikum gemäß Anspruch 14, **dadurch gekennzeichnet, dass** die teilbare Tablette eine Bruchrille, mehrere parallel zueinander angeordnete Bruchrillen oder eine Kreuzbruchrille aufweist.

16. Orales Schilddrüsentherapeutikum gemäß einem der voranstehenden Ansprüche, enthaltend neben weiteren pharmazeutisch annehmbaren Hilfs- und Trägerstoffen, 5 bis 60 Gew.-% Dextrine, 0,1 bis 10 Gew.-% höherkettige Partialglyceride, 0,01 bis 5 Gew.-% Levothyroxin und/oder Liothyronin oder deren pharmazeutisch verträgliche Salze, 10 bis 45 Gew.-% Calciumhydrogenphosphat und 10 bis 65 Gew.-% Mikrokristalline Cellulose.

## Claims

1. Oral thyroid therapeutic agent agent comprising at least one active compound and, as auxiliaries, dextrins and long-chain partial glycerides according to the DAB 1999 or NF 19 Pharmacopoeia, and also further pharmaceutically acceptable auxiliaries and carriers, one of the active compounds being levothyroxine or a pharmaceutically acceptable salt thereof or liothyronine or a pharmaceutically acceptable salt thereof or a mixture of the active compounds mentioned.

2. Oral thyroid therapeutic agent according to Claim 1, **characterized in that** the content of dextrins is from 5 to 60% by weight.

3. Oral thyroid therapeutic agent according to any of the preceding claims, **characterized in that** the content of dextrins is from 15 to 50% by weight.

4. Oral thyroid therapeutic agent according to any of the preceding claims, **characterized in that** the content of long-chain partial glycerides is from 0.1 to 10% by weight.

5. Oral thyroid therapeutic agent according to any of the preceding claims, **characterized in that** the content of long-chain partial glycerides is from 0.5 to 4% by weight.

6. Oral thyroid therapeutic agent according to any of the preceding claims, **characterized in that** it furthermore comprises, as auxiliary, calcium phosphate.

7. Oral thyroid therapeutic agent according to any of the preceding claims, **characterized in that** it furthermore comprises, as auxiliary, cellulose.

8. Oral thyroid therapeutic agent according to any of the preceding claims, **characterized in that** the active compound content is from 0.01 to 5% by weight.

9. Oral thyroid therapeutic agent according to Claim 1, **characterized in that** the active compound content is from 0.05 to 2.5% by weight.

10. Oral thyroid therapeutic agent according to Claim 1, **characterized in that** the active compound content is from 0.1 to 1% by weight.

11. Oral thyroid therapeutic agent according to any one of the preceding claims, **characterized in that** one of the active compounds is a thyroid hormone.

12. Oral thyroid therapeutic agent according to any of the preceding claims, **characterized in that** it comprises, as further active compound, iodine salts.

13. Oral thyroid therapeutic agent according to Claim 12, **characterized in that** the iodine salts are iodides or iodates of the alkali metals and mixtures thereof, in particular potassium iodide or potassium iodate.

14. Oral thyroid therapeutic agent according to any of the preceding claims in the form of a capsule, a nondivisible tablet or a tablet which can be divided once or more than once.

15. Oral thyroid therapeutic agent according to Claim 14, **characterized in that** the divisible tablet is scored, has several scores arranged in parallel or has a quartering score.

16. Oral thyroid therapeutic agent according to any of the preceding claims, comprising, in addition to further pharmaceutically acceptable excipients and carriers, from 5 to 60% by weight of dextrins, from 0.1 to 10% by weight of long-chain partial glycerides, from 0.01 to 5% by weight of levothyroxine and/or liothyronine or pharmaceutically acceptable salts thereof, from 10 to 45% by weight of calcium hydrogenphosphate and from 10 to 65% by weight of microcrystalline cellulose.

## Revendications

1. Agent thérapeutique oral de la glande thyroïde comprenant au moins un ingrédient actif et, en tant qu'auxiliaires, des dextrines et des glycérides partiels à longue chaîne conformément aux pharmacopées DAB 1999 ou NF 19, ainsi que des auxiliaires et des supports pharmaceutiquement acceptables supplémentaires, l'un des ingrédients actifs étant la lévothyroxine, ou un sel pharmaceutiquement acceptable de celle-ci, ou la liothyronine, ou un sel pharmaceutiquement acceptable de celle-ci, ou des mélanges desdits ingrédients actifs.

2. Agent thérapeutique oral de la glande thyroïde selon la revendication 1, **caractérisé en ce que** la teneur en dextrines est de 5 à 60 % en poids.

3. Agent thérapeutique oral de la glande thyroïde selon l'une quelconque des revendications précédentes, **caractérisé en ce que** la teneur en dextrines est de 15 à 50 % en poids.

4. Agent thérapeutique oral de la glande thyroïde selon l'une quelconque des revendications précédentes, **caractérisé en ce que** la teneur en glycérides partiels à longue chaîne est de 0,1 à 10 % en poids.

5. Agent thérapeutique oral de la glande thyroïde selon l'une quelconque des revendications précédentes, **caractérisé en ce que** la teneur en glycérides partiels à longue chaîne est de 0,5 à 4 % en poids.

6. Agent thérapeutique oral de la glande thyroïde selon l'une quelconque des revendications précédentes, **caractérisé en ce qu'**il comprend en outre du phosphate de calcium en tant qu'auxiliaire.

7. Agent thérapeutique oral de la glande thyroïde selon l'une quelconque des revendications précédentes, **caractérisé en ce qu'**il comprend en outre de la cellulose en tant qu'auxiliaire.

8. Agent thérapeutique oral de la glande thyroïde selon l'une quelconque des revendications précédentes, **caractérisé en ce que** la teneur en ingrédient actif est de 0,01 à 5 % en poids.

9. Agent thérapeutique oral de la glande thyroïde selon la revendication 1, **caractérisé en ce que** la teneur en ingrédient actif est de 0,05 à 2,5 % en poids.

10. Agent thérapeutique oral de la glande thyroïde selon la revendication 1, **caractérisé en ce que** la teneur en ingrédient actif est de 0,1 à 1 % en poids.

11. Agent thérapeutique oral de la glande thyroïde selon l'une quelconque des revendications précédentes, **caractérisé en ce que** l'un des ingrédients actifs est une hormone thyroïdienne.

12. Agent thérapeutique oral de la glande thyroïde selon l'une quelconque des revendications précédentes, **caractérisé en ce qu'**un ingrédient actif supplémentaire inclut des sels d'iode.

13. Agent thérapeutique oral de la glande thyroïde selon la revendication 12, **caractérisé en ce que** les sels d'iode sont des iodures ou des iodates des métaux alcalins ou leurs mélanges, en particulier, l'iodure de potassium ou l'iodate de potassium.

14. Agent thérapeutique oral de la glande thyroïde selon l'une quelconque des revendications précédentes, sous la forme d'une capsule, d'un comprimé non divisible ou d'un comprimé divisible une ou plusieurs fois.

15. Agent thérapeutique oral de la glande thyroïde selon la revendication 14, **caractérisé en ce que** le comprimé divisible présente une barre de cassure, plusieurs barres de cassure agencées parallèlement les unes aux autres ou une barre de cassure en croix.

16. Agent thérapeutique oral de la glande thyroïde selon l'une quelconque des revendications précédentes, comprenant, en plus d'auxiliaires et de supports pharmaceutiquement acceptables supplémentaires, de 5 à 60 % en poids de dextrines, de 0,1 à 10 % en poids de glycérides partiels à longue chaîne, de 0,01 à 5 % en poids de lévothyroxine et/ou de liothyronine, ou de leurs sels pharmaceutiquement acceptables, de 10 à 45 % en poids d'hydrogénophosphate de calcium et de 10 à 65% en poids de cellulose microcristalline.
